# EUROPÄISCHE PATENTSCHRIFT

(11) **EP 3 867 196 B1**
(45) Veröffentlichungstag und Bekanntmachung des Hinweises auf die Patenterteilung: **23.08.2023**
(21) Anmeldenummer: 19794103.2
(22) Anmeldetag: 17.10.2019
(51) Int. Cl.: C01B 17/88, C01B 17/90, C01B 17/94, C01B 21/38, C07C 201/16, C07C 201/08

(54) **VERFAHREN ZUR AUFARBEITUNG VON MISCHSÄURE UND ABWASSER AUS DER NITRIERUNG VON AROMATEN**
PROCESS FOR WORKUP OF MIXED ACID AND WASTEWATER FROM THE NITRATION OF AROMATICS
PROCÉDÉ DE TRAITEMENT FINAL D'ACIDE MIXTE ET D'EAUX USÉES À PARTIR DE LA NITRATION DE COMPOSÉS AROMATIQUES

(30) Priorität: 19.10.2018 DE 102018217955
(43) Veröffentlichungstag der Anmeldung: 25.08.2021
(73) Patentinhaber: PLINKE GmbH, 61348 Bad Homburg (DE)
(72) Erfinder: WINTERBAUER, Hansjürgen, 61191 Rosbach (DE)
(74) Vertreter: Weickmann & Weickmann PartmbB
(86) Internationale Anmeldenummer: PCT/EP2019/078207
(87) Internationale Veröffentlichungsnummer: WO 2020/079144

(56) Entgegenhaltungen:
- DE-A1- 19 512 114
- DE-A1-102017 110 084
- DE-T5-112013 001 622
- US-A- 4 091 042
- US-A- 5 963 878

## Beschreibung

Die Erfindung betrifft ein Verfahren zur Aufarbeitung von Abfallstromkomponenten aus der Nitrierung von Aromaten, bei dem die darin enthaltene Salpetersäure durch Reaktion mit einem Aromaten unter adiabatischen Bedingungen umgesetzt wird. Weiter betrifft die Erfindung eine Vorrichtung zur Durchführung des Verfahrens.

Bei der Herstellung von nitroaromatischen Verbindungen durch Nitrierung mit Mischsäure fallen verschiedene Abfallströme an. Dies ist zum einen die bei der Reaktion eingesetzte Mischsäure, die bei der Reaktion verdünnt wird, saures Waschwasser aus der Aufarbeitung der Rohnitroaromaten und dünne Salpetersäure, die bei der Abgasbehandlung zurückgewonnen wird.

Aus der DE 196 36 191 A1 ist bereits ein Verfahren zur Reinigung und Aufkonzentrierung von gebrauchten, verunreinigten Schwefelsäuren, die bei der Nitrierung von aromatischen Kohlenwasserstoffen in Gegenwart von Schwefelsäure anfallen, bekannt. Hier werden die wasserdampfflüchtigen Verbindungen unter Zersetzung der stickstoffhaltigen Verbindungen vollständig entfernt und die so gereinigte Schwefelsäure aufkonzentriert. Die verunreinigte Schwefelsäure wird vorerwärmt und im Gegenstrom mit den Brüden der ersten Aufkonzentrierstufe bei Drücken zwischen 200 und 1000 mbar von wasserdampfflüchtigen Verbindungen befreit. Die Schwefelsäure wird in eine erste Aufkonzentrierstufe geleitet, in der sie unter indirekter Wärmezufuhr bei gleichem Druck aufkonzentriert wird und die Schwefelsäure wird anschließend in einer ein- oder mehrstufigen Vakuumkonzentrierung bei einem Druck der kleiner ist als in der ersten Aufkonzentrierstufe auf 88 bis 97 Gew.-% aufkonzentriert.

Der aus der Nitrierung kommende Rohnitroaromat ist mit Resten der Nitriersäure sowie mit Nebenprodukten verunreinigt und muss mehrfach gewaschen werden. Zunächst werden die Säurerückstände durch die sogenannte "Saure Wäsche" entfernt. Das bei der "Sauren Wäsche" anfallende Abwasser enthält größere Mengen an Salpetersäure und Schwefelsäure, wie die EP 0 736 514 A1 am Beispiel des Dinitrotoluols zeigt.

Die DE10 2006 013 579 B3 beschreibt ein Verfahren zur Verminderung der Abwassermenge bei der Herstellung von Dinitrotoluol (DNT) und gleichzeitiger Optimierung der Abwasserqualität durch Reduzierung des Anteils an organischen Verunreinigungen, bei welchem die bei der Nitrierung anfallende Abfallsäure in einem ersten Schritt nach Vorwärmung auf 150 bis 170 °C unter atmosphärischen Druckbedingungen in einer Kolonne im Gegenstrom mit Wasserdampf gestrippt wird, der durch die Konzentrierung der aus der Kolonne ablaufenden Schwefelsäure erzeugt wird. Dabei wird eine Strippdampfmenge zwischen 0,25 und 10 Gew.-% bezogen auf die Abfallsäuremenge eingesetzt und dadurch am Kopf der Kolonne eine Salpetersäure mit 20 bis 40 Gew.-% HNO₃ erhalten. Die Salpetersäure wird dann direkt oder nach Aufkonzentrierung wieder in den Nitrierprozess zurückgefahren. Die vorgereinigte Abfallsäure aus der atmosphärischen Strippung wird in einem zweiten Schritt bei einem Vakuum zwischen 200 und 600 mbar in einer Kolonne im Gegenstrom mit Wasserdampf gestrippt, der durch die Konzentrierung der aus der Kolonne ablaufenden Schwefelsäure erzeugt wird, wobei eine Strippdampfmenge zwischen 5 und 10 % Gew.-% bezogen auf die zulaufende vorgereinigte Abfallsäuremenge eingesetzt und dadurch am Kopf der Kolonne eine Kondensat erhalten wird, das in der sauren Wäsche des DNT wieder eingesetzt werden kann. Die aus der Vakuumstrippung ablaufende Schwefelsäure wird in einer nachgeschalteten Schwefelsäurekonzentrierung ein- oder mehrstufig bei einem Vakuum zwischen 150 und 30 mbar, bevorzugt zwischen 100 und 50 mbar auf Konzentrationen zwischen 85 und 98 % H₂SO₄ aufkonzentriert und dabei ein Kondensat erhalten, das neben geringen Mengen an Schwefelsäure nur noch Spuren von nitroaromatische Verbindungen in Konzentrationen < 100 ppm enthält. Nitrose Abgase aus den einzelnen Prozessschritten werden durch Absorption des NOx im Gegenstrom mit Wasser gereinigt und dabei Salpetersäure zurück gewonnen, die dann direkt oder nach Aufkonzentrierung in den Nitrierprozess zurück gefahren wird.

Die EP 2 295 375 B1 beschreibt ein Verfahren zur Aufarbeitung von Abfallsäure aus der Herstellung von Nitroaromaten, insbesondere der Herstellung von Dinitrotoluol (DNT) oder Trinitrotoluol (TNT), zur Gewinnung von konzentrierter und gereinigter Schwefelsäure und Salpetersäure, wobei in einer ersten Stufe die vorgewärmte Abfallsäure, die neben bis zu 80 Masse-% Schwefelsäure und Wasser als weitere Bestandteile Salpetersäure (HNO3), Nitrosylschwefelsäure (als HNO₂) und Nitroorganika, insbesondere DNT und Mononitrotoluol (MNT), enthält, in einer Strippkolonne im Gegenstrom zu Dampf, der vom Boden der Strippkolonne durch Erhitzen der vorkonzentrierten Schwefelsäure erhalten wird, in mindestens eine dampfförmige Phase, die Salpetersäure mit oder ohne Nitroorganika enthält, und eine vorkonzentrierte Schwefelsäure getrennt wird. Hierbei wird in nachgeschalteten Verfahrensschritten (i) die vom Boden der Strippkolonne erhaltene vorkonzentrierte Schwefelsäure einer weiteren Reinigung zur Entfernung von Nitroorganika und für höhere Konzentrationen zugeführt, und (ii) die aus der dampfförmigen Salpetersäurephase erhaltene Salpetersäure und die Nitroorganika, einschließlich der bei der weiteren Reinigung und Konzentration der vorkonzentrierten Schwefelsäure erhaltenen Nitroorganika, aufbereitet und in den Nitrierprozess zurückgeführt werden. Dieses Verfahren ist ferner dadurch gekennzeichnet, dass in der ersten Verfahrensstufe neben dem Strippen der vorgewärmten Abfallsäure in einer Strippkolonne im Gegenstrom zum Dampf aus der Schwefelsäureanreicherung (V1) eine Konzentration der im Strippdampf vorhandenen Salpetersäure im Gegenstrom zu zusätzlicher gereinigter und gegebenenfalls frischer konzentrierter Schwefelsäure mit einer Konzentration im Bereich von 75 bis 97 Masse-% und vorzugsweise 80 bis 96 Masse-% durchgeführt wird. Die Salpetersäuredämpfe, die von der Oberseite der Kolonne der ersten Stufe erhalten werden, werden zu einer Salpetersäure direkt in hochkonzentrierter Form kondensiert, die für die Rückführung in den Nitrierprozess geeignet ist.

Die US 4 496 782 A beschreibt Verfahren zur Rückgewinnung von Salpetersäure aus der verbrauchten Säurephase einer gemischten sauren Mononitrierungsreaktion umfassend die Zugabe einer ausreichenden Menge Salpetersäure, um mindestens etwa 2 Gew.-% Salpetersäurekonzentration in der verbrauchten Säurephase aus der Mononitrierungsreaktion bereitzustellen. Durch adiabatische Umsetzung eines mononitroaromatischen Kohlenwasserstoffs in mehr als einer stöchiometrischen Menge mit der Salpetersäure in der verbrauchten Säurephase, wird dann ein dinitroaromatisches Kohlenwasserstoffprodukt und eine Salpetersäurekonzentration von weniger als etwa 0,25 Gew.-% in der verbrauchten Säurephase erhalten.

Die US 4 650 912 A beschreibt ein Verfahren zur Denitrifikation der Schwefelsäure- und Salpetrige Säure-haltigen verbrauchten Säurephase aus der Nitrierung eines aromatischen Kohlenwasserstoffs nach dem Mischsäureverfahren, umfassend die Bildung eines Denitrifikationsreaktionsmediums durch Kontaktieren der verbrauchten Säurephase mit einem aromatischen Kohlenwasserstoff unter Nitrierreaktionsbedingungen zur Gewinnung der Salpetersäure durch Bildung eines nitroaromatischen Kohlenwasserstoffs. Hierbei wird eine Menge an aromatischem Kohlenwasserstoff zugegeben, die geringfügig kleiner oder gleich der stöchiometrischen Menge ist, die erforderlich ist, um die verbrauchte saure Phase der Salpetersäure abzubauen und das Denitrifikationsreaktionsmedium auf das Auftreten einer dunkelroten bis schwarzen Farbe photometrisch überwacht, woraufhinbeim Nachweis einer solchen Farbe das molare Verhältnis von aromatischem Kohlenwasserstoff zu Salpetersäure im Denitrifikationsreaktionsmedium angepaßt wird, um die Farbe zu eliminieren.

Die US 8 907 144 B2 beschreibt ein Verfahren zur kontinuierlichen adiabatischen Nitrierung von Toluol zu Mononitrotoluol (MNT). Das Verfahren ergibt eine Produktqualität von MNT, die mit der einer isothermen Produktion vergleichbar ist. Das Verfahren verwendet überschüssiges Toluol, wobei die Reaktionsgeschwindigkeit so gesteuert wird, dass ein Rest von 0,003-0,102 Gew.-% Salpetersäure in der verbrauchten Säure und eine orange-rote Farbe der verbrauchten Säure erhalten bleibt. Weitere Prozessbedingungen sind rückkonzentrierte Schwefelsäure bei 83 bis 99 °C mit einer Konzentration an Schwefelsäure von 66 bis 70,5 Gew.-%. Diese wird mit Salpetersäure zu einer Mischsäure mit 1,0 bis 3,8 Gew.-% Salpetersäure vermischt und Toluol wird mit einer Menge von 1,1 bis 1,71 Mol Toluol/Mol Salpetersäure zugegeben.

Obwohl die in US 4 496 782 A und die US 4 650 912 A beschriebenen Verfahren, da sie die Salpetersäure umsetzen und diese dadurch nicht rückkonzentriert werden muss, wesentlich günstiger sein müssten als die Verfahren mit Strippung, haben sie sich nicht durchgesetzt. Beide Verfahren geben die aromatische Verbindung nur im unterstöchiometrischen bis maximal stöchiometrischen Verhältnis zur Salpetersäure zu, so dass keine vollständige Umsetzung der Salpetersäure erfolgt. In US 4 650 912 A wird sogar das Auftreten einer dunkelroten bis schwarzen Färbung beschrieben, die bei einer überstöchiometrischen Zugabe der Aromaten auftritt und einen sicheren kontinuierlichen Betrieb dieser Verfahren erschwert. Auch beschränken sich beide Verfahren auf die Aufarbeitung der Mischsäure aus der Nitrierreaktion und die anderen salpetersäurehaltigen Abfallströme müssen separat behandelt werden. Bei dem in US 8 907 144 B2 beschriebenen Verfahren zur adiabatischen Nitrierung wird zwar Toluol im stöchiometrischen Überschuss zugegeben, aber auch hier wird die Reaktion so gesteuert, dass nicht die gesamte Salpetersäure umgesetzt wird. Auch handelt es sich dabei nicht um ein Verfahren zur adiabatischen Herstellung von MNT und nicht um ein Verfahren zur Aufarbeitung der Abfallsäure aus der isothermen Nitrierung von Aromaten.

Folgende weitere Dokumente bilden technologischen Hintergrund zur vorliegenden Erfindung:
DE 11 2013 001 622 T5 beschreibt ein Verfahren zur adiabatischen Herstellung von Mononitrotoluol, gibt aber keinerlei Hinweise für eine Behandlung der anfallenden Abfallkomponenten der Mischsäure und Abwasser. In ähnlicher Weise offenbaren die US-Patente US 5,963,878 A und US 4,091,042 A, sowie die DE 10 2017 110 084 A1 Verfahren zur Nitrierung von Aromaten, sie beschäftigen sich jedoch nicht mit der Aufarbeitung von entstehenden Mischsäuren oder Abwässern. Die DE 195 12 114 A1 offenbart ein Verfahren zum Entfernen und Wiedergewinnen von Salpetersäure, Schwefelsäure und Stickoxiden aus bei der Nitrierung von Toluol oder Mononitrotoluolen nach Abtrennung der Nitriersäure anfallenden rohen Dinitrotoluolen. Die Aufarbeitung von Mischsäure und Abwasser aus der Nitrierung von Aromaten behandelt auch diese Literaturstelle nicht.

Dem erfindungsgemäßen Verfahren liegt nun die Aufgabe zugrunde, effektiv bei der Nitrierung von Aromaten anfallende salpetersäurehaltige Abfallströme aufzuarbeiten. Bevorzugt sollen alle entsprechenden Komponenten, nämlich die eingesetzte Mischsäure, die bei der Reaktion verdünnt wird, das saure Waschwasser aus der Aufarbeitung der Rohnitroaromaten und die dünne Salpetersäure, die bei der Abgasbehandlung anfällt, gemeinsam aufgearbeitet werden.

Gelöst wird diese Aufgabe erfindungsgemäß durch ein Verfahren zur Aufarbeitung von Abfallströmen aus der Nitrierung von Aromaten, bei dem darin enthaltene Salpetersäure durch Reaktion mit einem Aromaten unter adiabatischen Bedingungen umgesetzt wird, welches dadurch gekennzeichnet ist, dass
a) mindestens eine Abfallstromkomponente ausgewählt aus bei der Nitrierung anfallender Abfallsäure (Mischsäure), saurem Waschwasser aus der Aufarbeitung von Rohnitroaromaten und dünner Salpetersäure, die bei einer Abgasbehandlung im Verlauf der Nitrierung anfällt, bereitgestellt wird,
b) die mindestens eine Abfallstromkomponente mit rückkonzentrierter Schwefelsäure gemischt wird,
c) ein Aromat in stöchiometrischem Überschuss bezogen auf die Salpetersäure in die Mischung eindosiert wird,
d) die erhaltene Reaktionsmischung in einem adiabatisch betriebenen Reaktor umgesetzt wird,
e) in einem Separator die erhaltene organische Phase von der schwefelsauren Phase getrennt wird,
f) die schwefelsaure Phase im Vakuum aufkonzentriert wird, und
g) zumindest ein Teilstrom der rückkonzentrierten Schwefelsäure aus Schritt g) in Schritt b) eingesetzt wird.

In einer bevorzugten Ausführungsform der Erfindung werden mindestens zwei Abfallstromkomponenten und besonders bevorzugt alle oben genannten Abfallstromkomponenten in Schritt a) vorgemischt und dann mit der rückkonzentrierten Schwefelsäure gemischt.

Das erfindungsgemäße Verfahren ermöglicht es, die salpeterhaltigen Abfallströme aus Nitrierverfahren vollständig und vorzugsweise auch gemeinsam durch adiabatische Nitrierung und Umsetzung mit einem Aromaten oder einem Nitroaromaten vollständig von Salpetersäure zu befreien und dabei die Reaktionsbedingungen und den Reaktor so zu gestalten, dass die bei ähnlichen Verfahren beschreibenden Probleme nicht auftreten und das Verfahren auch im praktischen Betrieb Bestand hat. Dies lässt sich überraschenderweise einfach durch Einsatz eines kontinuierlich betriebenen Reaktors und Rückführung eines Teilstroms der rückkonzentrierten, gereinigten Schwefelsäure erreichen. Mit dieser Fahrweise ist es überraschenderweise möglich, die aromatische Verbindung in stöchiometrischen Überschuss bezogen auf die in der Mischung enthaltene Salpetersäure einzusetzen, wodurch die Salpetersäure vollständig abreagieren kann. Die in US 4 650 912 A beschriebenen Probleme bei überstöchiometrischer Dosierung werden bei dem erfindungsgemäßen Verfahren überraschenderweise nicht beobachtet.

Im Folgenden wird das erfindungsgemäße Verfahren am Beispiel der Nitrierung von Toluol zu Dinitrotoluol beschrieben. Alle Druckangeben sind als Absolutdruck angegeben. Das Verfahren ist natürlich auch auf alle anderen aromatischen Verbindungen, die entweder einfach, doppelt oder auch mehrfach nitriert werden können, anwendbar. Beispiele hierfür wären Nitrierungen von Benzol zu Nitrobenzol oder Dinitrobenzol, von Toluol zu Mononitro(MNT)-, Dinitro(DNT)- oder Trinitrotoluol (TNT), von Nitrochlorbenzol (NCB) zu Mononitrochlorbenzol (MNCB) oder Dinitrochlorbenzol (DNCB), etc. Die angegebenen Aromaten stehen hier nur als Beispiele und schränken den Anwendungsbereich des erfindungsgemäßen Verfahrens nicht ein, sodass alle Angaben und bevorzugten Ausführungsformen auch auf die Nitrierung anderer Aromaten zutreffen.

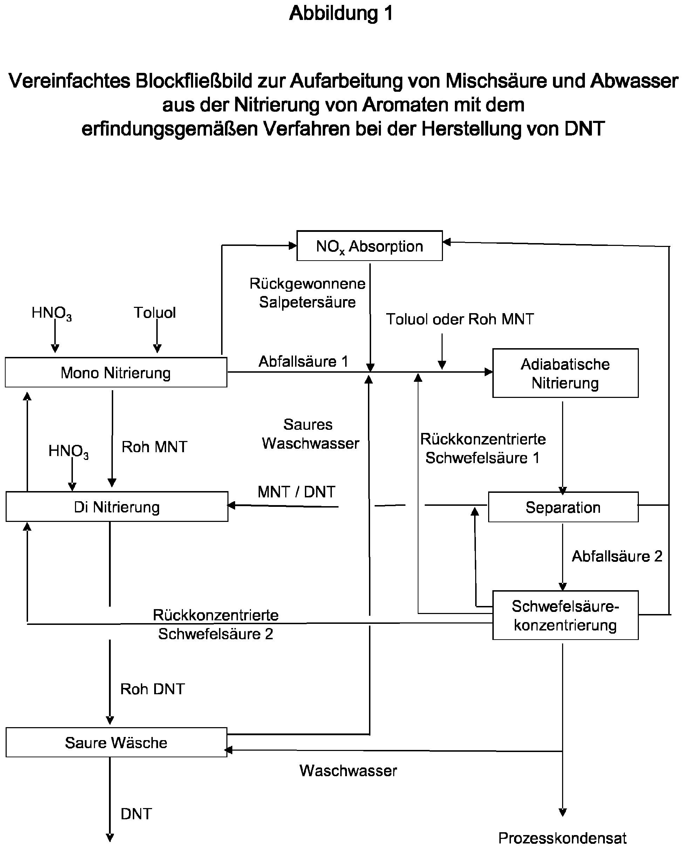

**Abbildung 1** zeigt ein vereinfachtes Blockfließbild für ein Verfahren zur Herstellung von DNT mit Säureaufarbeitung nach dem erfindungsgemäßen Verfahren. In der Mono-Nitrierung wird Toluol unter Zugabe von Salpetersäure und Schwefelsäure aus der Di-Nitrierung zu Mononitrotoluol MNT nitriert. Das erhaltene Roh-MNT, ein Gemisch der verschiedenen Isomere mit einem Anteil an DNT, wird dann in der Di-Nitrierung unter weiterer Zugabe von Salpetersäure und rückkonzentrierter Schwefelsäure zu DNT weiternitriert. Das dabei erhaltene Roh-Dinitrotoluol wird in einer sauren Wäsche weitestgehend von anhaftender Salpetersäure und Schwefelsäure befreit.

Erfindungsgemäß bevorzugt wird die Abfallsäure 1, die aus der Mono-Nitrierung anfällt, mit dem sauren Waschwasser aus der sauren Wäsche und der rückgewonnenen Salpetersäure aus einer bei Nitrierungen üblicherweise eingeschlossenen NOₓ Absorption gemischt. Die Mischung kann erfindungsgemäß in einem einfachen Behälter erfolgen. Alternativ können aber auch Mischapparate entsprechend dem Stand der Technik wie Rührapparate, statische Mischer oder ähnliche eingesetzt werden.

Die Mischung wird vorzugsweise unter Rückgewinnung der Energie der bei der Schwefelsäurekonzentrierung erhaltenen rückkonzentrierten Schwefelsäure 2, die dabei gekühlt wird, aufgewärmt. Aufwärmung kann aber auch durch indirekte Dampfbeheizung oder einer Kombination aus Dampfbeheizung und Energierückgewinnung erfolgen. Die Vorwärmung der Mischung wird so eingestellt, dass die Temperatur nach Zugabe der rückkonzentrierten Schwefelsäure 1 im Bereich 70 °C bis 130 °C liegt. Dann erfolgt die Zugabe ungekühlter rückkonzentrierter Schwefelsäure 1. Die rückkonzentrierte Schwefelsäure 1, die zur adiabatischen Nitrierung gefahren wird, kann erfindungsgemäß die gleiche oder eine geringere Konzentration aufweisen als die rückkonzentrierte Schwefelsäure 2, die in der Di-Nitrierung eingesetzt wird. Die Menge und Konzentration der rückkonzentrierten Schwefelsäure 1 wird dabei erfindungsgemäß so gewählt, dass sich eine Mischsäure mit einem Schwefelsäuregehalt zwischen 60 % und 70 % H₂SO₄ und einem Salpetersäuregehalt zwischen 1 % und 5 % HNO₃ ergibt. Die benötigte Konzentration und Temperatur in der Konzentrationsstufe lassen sich über das bei der Konzentrierstufe eingestellte Vakuum festlegen. Der Fachmann kann hierzu die gängige Literatur zu Stoffwerten der Schwefelsäure, wie beispielsweise Perry's Chemical Engineers` Handbook (McGraw Hill), nutzen.

Soweit in einer Anfahrphase der Reaktion nicht aus einer vorhergehenden Verfahrensführung rückkonzentrierte Schwefelsäure vorhanden ist, kann anstelle dieser frische Schwefelsäure entsprechender Konzentration eingesetzt werden.

Die Temperatur am Reaktoreintritt wird im Bereich 70 °C bis 130 °C, bevorzugt im Bereich 90°C bis 110 °C eingestellt, so dass die Reaktion direkt nach Zugabe der aromatischen Verbindung einsetzt. Über ein spezielles Dosiersystem wird der Aromat, beispielsweise Toluol oder MNT, für die Nitrierreaktion in die umlaufende Schwefelsäure-Salpetersäure-Wasser-Mischung dosiert und fein verteilt. Alternativ zu reinem MNT als einzelnes Isomer oder als Isomerengemisch kann hier auch Roh-MNT aus der Mono Nitrierung, das einen Anteil DNT enthält, eingesetzt werden. In der adiabatischen Nitrierung erfolgt dann in einem adiabatisch betriebenen Reaktor die quasi vollständige Umsetzung der in der Mischung vorhandenen Salpetersäure mit dem Aromaten zum Nitroaromaten. Bei Toluol erhält man hier überwiegend MNT als Isomerengemisch; bei Einsatz von MNT vorwiegend DNT bei der Reaktion.

Als Reaktor wird bevorzugt ein Rohrreaktor eingesetzt. Rührreaktoren wären erfindungsgemäß auch möglich. Der Rohrreaktor hat aber den Vorteil, dass er wesentlich weniger Platz benötigt. Der Rohrreaktor ist bevorzugt modular aufgebaut und enthält statische Mischelemente für die Wiedervermischung der Reaktionsmedien. Die Anzahl der statischen Mischelemente liegt erfindungsgemäß zwischen 2 und 20 Stück. Durch die Mischelemente ergibt sich ein Druckverlust über den Reaktor. Der Betriebsdruck am Reaktoreintritt liegt erfindungsgemäß im Bereich 1 bar bis 10 bar, bevorzugt im Bereich 3 bar bis 6 bar.

Der Aromat wird im stöchiometrischen Überschuss im Bereich 1 % - 20 % bevorzugt 3 % - 10 % bezogen auf die Salpetersäuremenge zugegeben, um eine vollständige Reaktion zu gewährleisten. Höherer Überschuss wäre zwar auch möglich und würde die Reaktion nicht stören, ist aber nicht angestrebt, da die organischen Substanzen im nächsten Schritt wieder abgetrennt werden.

Nach Umsetzung der Salpetersäure im adiabatischen Reaktor werden die Schwefelsäure und die organische Produktphase separiert. Dies erfolgt bevorzugt unter Ausnutzung der unterschiedlichen Dichten der Schwefelsäure und des erhaltenen Nitroaromaten, hier beispielsweise ein Toluol-MNT- bzw. ein MNT-DNT-Gemisch. Als Separatoren können einfache Behälter entsprechend dem Stand der Technik mit oder ohne Einbauten oder aber auch Zentrifugen zum Einsatz kommen. Der Druck im Separator beträgt erfindungsgemäß bevorzugt zwischen 1 bar und 2 bar. Dadurch wird verhindert, dass die Produktmischung aus Aromat bzw. Nitroaromat teilweise verdampft und sich durch die Verdampfung die Separation erschwert. Der Druckverlust über den Reaktor entspricht der eingetragenen Mischungsenergie. Die abgetrennte organische Phase kann dann separat oder zusammen mit den bei der Schwefelsäurekonzentrierung zurückgewonnenen organischen Verbindungen in die Mono und/oder Di-Nitrierung zur weiteren Nitrierung gefahren oder alternativ direkt aufgearbeitet werden.

Überraschenderweise wurden die Farberscheinungen, wie sie in der US 4 650 912 A bei überstöchiometrischer Zugabe des Aromaten beschrieben sind, bei dem erfindungsgemäßen Verfahren nicht beobachtet. Dies könnte durch die erhöhte Temperatur und die unterschiedliche Säurezusammensetzung, die sich durch die Rückführung gereinigter rückkonzentrierter Schwefelsäure ergibt, bedingt sein.

Die separierte Abfallschwefelsäure 2 hat durch die adiabatische Fahrweise die Reaktionsenergie aufgenommen und sich dadurch erfindungsgemäß um 10 bis 40°C erhitzt. Die Abfallsäure 2 wird in einen Verdampfer, der bevorzugt bei einem Druck wischen 30 mbar und 500 mbar betrieben wird, geflasht und dadurch vorkonzentriert. Die weitere Konzentrierung der Schwefelsäure erfolgt mit den üblichen Apparaten zur Konzentrierung von Schwefelsäure entsprechende dem Stand der Technik und kann abhängig von der Leistung der Anlage ein- oder mehrstufig erfolgen. Falls erforderlich, kann auch entsprechend dem Stand der Technik organisches Lösungsmittel in das Kondensationssystem der Schwefelsäurekonzentrierung eindosiert werden, um Ablagerungen von Nitroaromaten mit höherem Schmelzpunkt zu vermeiden. Die rückkonzentrierte Schwefelsäure 1 wird zur adiabatischen Nitrierung zurückgefahren. Die verbleibende Schwefelsäure kann gegebenenfalls mit den Verfahren nach dem Stand der Technik weiterkonzentriert werden und wird als rückkonzentrierte Schwefelsäure 2 zur Di-Nitrierung gefahren. Die Weiterkonzentrierung erfolgt bei einem Vakuum zwischen 150 und 30 mbar, bevorzugt zwischen 100 und 50 mbar, auf die gewünschte Endkonzentration zwischen 85 und 98 % H₂SO₄. Je nachdem, welche Konzentration bei dem jeweiligen Nitrierverfahren eingesetzt wird, kann jede beliebige für das Nitrierverfahren benötigte Schwefelsäurekonzentration für die rückkonzentrierte Schwefelsäure 2 eingestellt werden. Die angegebenen Konzentrationen sind nur exemplarisch gewählt und sollen das Verfahren nicht einschränken.

Das Prozesskondensat aus der Schwefelsäurekonzentrierung kann teilweise zur sauren Wäsche eingesetzt werden. Alle in den Prozessschritten anfallenden Abgase werden zur NOₓ-Absorption geleitet und die enthaltenen nitrosen Gase als Salpetersäure zurückgewonnen. Die dabei erhaltene Salpetersäure wird erfindungsgemäß mit der Abfallsäure 1 gemischt und zur Umsetzung in die adiabatische Nitrierung gefahren.

Das erfindungsgemäße Verfahren bietet gegenüber dem Stand der Technik den Hauptvorteil, dass die gesamte Salpetersäure umgesetzt wird und nicht rückkonzentriert werden muss. Bei den heutzutage eingesetzten Verfahren entsprechend DE 10 2006 013 579 B3 bzw. EP 2 295 375 B1, bei denen die Salpetersäure aus der Abfallschwefelsäure ausgestrippt wird, wird für dieses Ausstrippen und das anschließende Konzentrieren der Salpetersäure Energie benötigt. Bei dem erfindungsgemäßen Verfahren hingegen ist keine Energiezufuhr erforderlich, im Gegenteil, durch die adiabatische Fahrweise wird sogar die freiwerdende Reaktionsenergie noch für die Rückkonzentrierung der Schwefelsäure nutzbar gemacht. Auch werden beim erfindungsgemäßen Verfahren keine Kolonnen für die Strippung benötigt, wodurch das für die Aufstellung der Vorrichtung zur Durchführung des Verfahrens benötigte Gebäude wesentlich niedriger gebaut werden kann und damit die Investitionskosten entsprechend sinken.

Eine Vorrichtung zur Durchführung des erfindungsgemäßen Verfahrens wird im Folgenden beschrieben, diese Vorrichtung ist aber kein Gegenstand der vorliegenden Erfindung.

Die Vorrichtung besteht aus einer Mischeinheit, in der die Abfallsäure aus der Nitrierung, die rückgewonnene dünne Salpetersäure aus der Absorption von nitrosen Gasen im Prozessverlauf und das saure Waschwasser aus der sauren Wäsche der Nitroaromaten gemischt werden. Ferner umfasst die Vorrichtung mindestens einen Wärmeaustauscher zum Vorwärmen und der erhaltenen Mischung, welche durch das erfindungsgemäße Verfahren aufgearbeitet werden soll, eine Pumpe mit welcher diese Mischung mit Schwefelsäure, insbesondere eine rückkonzentrierte Schwefelsäure aus dem erfindungsgemäßen Verfahren vermischt wird und welche den am Reaktoreintritt benötigten Druck erzeugen kann. Ferner umfasst die Vorrichtung eine Injektionseinheit, in der der zu nitrierende Aromat in die Mischung über Düsen eindosiert und fein verteilt wird. Die Vorrichtung umfasst des Weiteren einen modular aufgebauten Rohrreaktor mit zwischen 2 und 20 statischen Mischern, in welchem die Nitrierung des Aromaten erfolgt, sowie einen Separator der dem Rohrreaktor nachgeschaltet ist und in dem das aus dem Rohrreaktor austretende Gemisch in eine organische Phase und eine Säurephase getrennt wird. Des Weiteren umfasst die erfindungsgemäße Vorrichtung einen Flashverdampfer und eine Konzentriereinheit mit einem indirekt beheizten Wärmeaustauscher und einem dazu gehörigen Verdampfer mit zugehöriger Brüdenkondensation sowie eine Vakuumeinheit.

Vorteilhafterweise ist zusätzlich eine ein- oder mehrstufige Schwefelsäurekonzentrierung installiert, in der restliche Schwefelsäure, die nicht zur adiabatischen Nitrierung gefahren wird, weiter konzentriert wird.

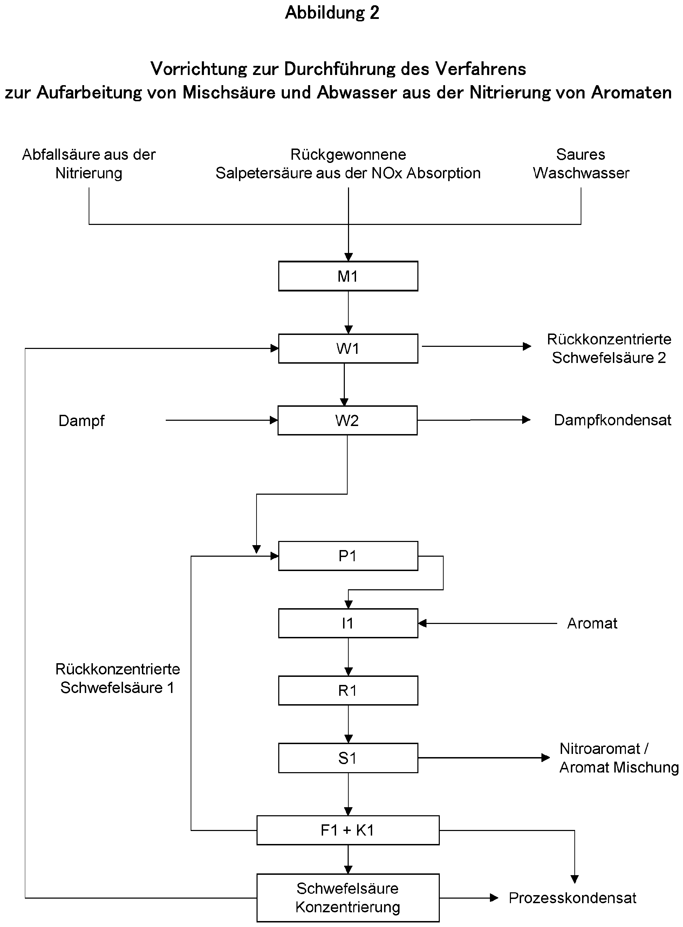

**Abbildung 2** zeigt den vereinfachten Aufbau einer Vorrichtung zur Durchführung des erfindungsgemäßen Verfahrens. Die Vorrichtung besteht aus einer Mischeinheit M1 in der die Abfallsäure aus der Nitrierung, die rückgewonnene Salpetersäure aus der NOₓ-Absorption und das saure Waschwasser aus der Nitroaromatenwäsche gemischt werden. Als Mischeinheit M1 kann ein einfacher Tank ohne Einbauten dienen. Alternativ kann auch ein Tank mit Rührvorrichtung oder eine Apparatur mit statischen Mischern eingesetzt werden.

Die Mischung wird anschließend in einem Wärmeaustauscher W1, vorzugsweise durch indirekten Wärmeaustausch mit der aus der Schwefelsäurekonzentrierung ablaufenden rückkonzentrierten Schwefelsäure 2 und mit einem indirekt mit Dampf beheizten Wärmetauscher W2 vorgewärmt. Optional kann auch nur einer der beiden Vorwärmer eingesetzt werden. Die vorgewärmte Mischung wird saugseitig der Pumpe P1 zusammen mit rückkonzentrierter Schwefelsäure aus der der Flashung F1 und Konzentrierung K1 zugegeben. Die Pumpe P1 vermischt die beiden Ströme und erzeugt den am Reaktoreintritt benötigten Druck der Flüssigkeit. Die Mischung durchströmt die Injektionseinheit I1 in der der zu nitrierende Aromat in die Mischung über Düsen eindosiert und fein verteilt wird. Die Reaktionsmischung fließt anschließend durch den Rohrreaktor R1. Dieser ist modular aufgebaut und besteht aus Rohrleitungsteilen unterschiedlicher Länge. Zwischen den Rohrleitungsteilen sind erfindungsgemäß zwischen 2 und 20 statische Mischer eingebaut, die für die entsprechende Wiedervermischung der Reaktionsmischung sorgen. Nach dem Reaktor R1 folgt ein Separator S1, in dem die organische Phase von der Säurephase getrennt wird. Die erhaltene Schwefelsäurephase wird dann in einen Flashverdampfer F1 geleitet, der im Vakuum betrieben wird. Die relative Aufstellung des Separators S1 zu F1 wird so gewählt, dass bei dem eingestellten Vakuum die Säurephase aufgrund des unterschiedlichen Druckes selbständig von S1 zu F1 überläuft. Hier verdampft spontan durch die Flashverdampfung Wasser, die in der Schwefelsäure noch entsprechend der Löslichkeit enthaltenen organischen Bestandteile und HNO₂ aus der Schwefelsäure ab bis diese auf Siedetemperatur entsprechend dem eingestellten Vakuum abgekühlt ist. Anschließend oder alternativ integriert in den Flashverdampfer erfolgt eine indirekte Beheizung der Schwefelsäure und Konzentrierung auf die gewünschte Konzentration der rückkonzentrierten Schwefelsäure 1 mit der Konzentriereinheit K1 die bei Kombination mit F1 nur aus einem entsprechenden indirekt beheizten Wärmeaustauscher besteht. Bei einer an F1 anschließenden separaten Konzentrierung besteht K1 aus einem indirekten beheizten Wärmeaustauscher und einem dazugehörigen Verdampfer. Der für adiabatische Nitrierung benötigte Teil der rückkonzentrierten Schwefelsäure aus der Flashung und Konzentrierung F1 + K1 wird zur Saugseite der Pumpe P1 geführt. Die bei der Verdampfung in F1 + K1 erhaltenen Wasserdampfbrüden werden entweder separat oder zusammen mit den aus der anschließenden Schwefelsäurekonzentrierung kommenden Brüden kondensiert (Prozesskondensat). Inertgase werden von einer Vakuumeinheit abgezogen. Als Vakuumeinheit wird vorzugsweise eine indirekt gekühlte Vakuumpumpe eingesetzt, da sowohl eine Kühlung durch direkte Wasserzuführung als auch der Einsatz von Dampf-betriebenen Vakuumstrahlern die Gesamtabwassermenge erhöhen würde.

Die restliche Schwefelsäure, die nicht zur adiabatischen Nitrierung gefahren wird, wird in eine angeschlossene Schwefelsäurekonzentrierung gefahren. In dieser wird die Säure entsprechend dem Stand der Technik ein- oder mehrstufig bei Vakuum aufkonzentriert. Als Vakuumeinheit wird auch hier vorzugsweise eine (bzw. leistungsbedingt auch mehrere) indirekt gekühlte Vakuumpumpe eingesetzt, da sowohl eine Kühlung durch direkte Wasserzuführung als auch der Einsatz von Dampf betriebenen Vakuumstrahlern die Gesamtabwassermenge erhöhen würde. Wenn F1+K1 und die Schwefelsäurekonzentrierung bei gleichem Vakuum betrieben werden, kann auch eine gemeinsame Vakuumeinheit genutzt werden. Alle Abgase aus den einzelnen Prozessschritten werden zur NOₓ-Absorption geleitet um evtl. enthaltene nitrose Abgase als Salpetersäure zurückzugewinnen.

Zum Beheizen und Konzentrieren der Säure kommen die gängigen Verdampfertypen wie beispielsweise Naturumlaufverdampfer, Zwangsumlaufverdampfer, Horizontalverdampfer, etc. zum Einsatz. Entsprechende Verfahren zur Schwefelsäurekonzentrierung sind hinreichend bekannt und werden hier nicht näher erläutert. Als Werkstoffe für den Reaktor R1, den Separator S1, den Flashverdampfer F1, den Verdampfer bei K1 und die Verdampfer in der Schwefelsäurekonzentrierung werden korrosionsbeständige Materialien, wie beispielsweise Email oder PTFE - ausgekleideter Stahl eingesetzt. Für dampfbeheizte Wärmeaustauscher wie W2, den Heizer in K1 und die Heizer in der Schwefelsäurekonzentrierung kommen korrosionsbeständige Materialien wie beispielsweise Tantal zum Einsatz, wie sie für die Konzentrierung von Schwefelsäure üblicherweise eingesetzt werden. Für Säure-Säure-Wärmeaustauscher wie W1 werden Materialien wie Siliziumcarbid oder Tantal eingesetzt. Als Werkstoffe für Rohrleitungen in Kontakt mit heißer Schwefelsäure werden korrosionsbeständigem Material wie beispielsweise Email oder PTFEausgekleideter Stahl eingesetzt. Für Apparate und Maschinen in Kontakt mit Prozesskondensat werden geeignete Edelstähle eingesetzt.

Die folgenden Beispiele erläutern die Erfindung weiter.

**Beispiel 1** zeigt exemplarisch, wie sich die einzelnen Massenströme zueinander verhalten können. Die einzelnen Massenströme können natürlich auch in ihrem Mengenverhältnis sowie in ihrer Zusammensetzung variieren, je nachdem, aus welchem Prozess die Massenströme kommen und wie die jeweilige Nitrierung, Wäsche und NOₓ Absorption betrieben werden. Das Beispiel 1 soll das erfindungsgemäße Verfahren näher beschreiben, die angegebenen Werte sollen aber das Verfahren nicht einschränken. Alle %-Angaben beziehen sich auf Gewichtsprozent.

Bei einem Nitrierprozess zur Herstellung von DNT fallen 9'859 kg/h Abfallsäure mit folgender Zusammensetzung an: 71 % H₂SO₄, 0,8 % HNO₃, 1,6 % HNO₂, 0,5 % DNT, 26,1 % H₂O. Weiterhin fallen 2`260 kg/h Waschwasser mit folgender Zusammensetzung an: 8 % H₂SO₄, 17 % HNO₃, 1 % HNO₂, 1 % DNT, 73 % H₂O. Aus der NOₓ Absorption werden 811 kg/h Salpetersäure mit 55 % HNO₃ und 45 % H₂O zurückgewonnen. Die 3 Ströme werden gemischt und auf 90 °C vorgewärmt. Anschließend werden zu der Mischung 16'086 kg/h ungekühlte rückkonzentrierte Schwefelsäure 1 mit 75 % H₂SO₄ und 25 % Wasser mit ca. 112 °C zugegeben, was der Siedetemperatur der Schwefelsäure in der zugehörigen Rückkonzentrierstufe bei 80 mbar entspricht. In die sich ergebende Mischung, die dann eine Temperatur von ca. 102 °C hat, werden 1'462 kg/h Toluol eingespritzt und die Mischung in den adiabatischen Rohrreaktor geleitet. Im adiabatischen Rohrreaktor wird die Mischung durch aufeinanderfolgende statische Mischer weiter durchmischt, um hier eine vollständige Reaktion zu erreichen. Bei Beispiel 1 wurden 10 Mischelemente eingesetzt. Am Austritt des adiabatischen Reaktors erhält man eine Mischung mit 63,14 % H₂SO₄, 0,59 % HNO₂, 6,45 % MNT, 0,24 % DNT, 0,44 % Toluol und 29,1 % H₂O. HNO₃ ist abreagiert und wenn überhaupt nur noch in Spuren vorhanden. Die Temperatur der Reaktionsmischung hat sich durch die frei werdende Reaktionsenergie und die adiabatische Reaktionsführung auf ca. 130 °C erhöht. Die Reaktionsmischung wird in einen Separator geleitet. An der Oberfläche scheidet sich die organische Phase ab und wird abgezogen. Die Schwefelsäurephase wird dann in einem Verdampfer bei 80 mbar gefälscht und durch die resultierende Wasserverdampfung entsprechend vorkonzentriert. Anschließend wird die Säure ebenfalls bei 80 mbar auf 75 % H₂SO₄ durch indirekte Beheizung nach den gängigen Methoden zur Schwefelsäurekonzentrierung entsprechend dem Stand der Technik weiter konzentriert. Bei der Konzentrierung der Schwefelsäure werden die gelösten organischen Verbindungen sowie enthaltene HNO₂ zusammen mit dem Wasser aus der Schwefelsäure ausgedampft. Die Dämpfe werden kondensiert und die organische Phase wird von der erhaltenen wässrigen Phase entsprechend dem Stand der Technik getrennt. Um Ablagerungen von DNT zu vermeiden, wird in das Kondensatsystem entsprechend dem Stand der Technik ein Lösungsmittel, hier MNT zugegeben. Die verbleibende, nicht zur adiabatischen Nitrierung zurückgefahrene Schwefelsäure wird im Vakuum von 80 mbar durch indirekte Beheizung mit Dampf, nach den gängigen Methoden zur Schwefelsäurekonzentrierung entsprechend dem Stand der Technik weiter bis auf 93 % H₂SO₄ konzentriert. Diese rückkonzentrierte Schwefelsäure 2 wird dann gekühlt, wobei ein Teil der Energie wie oben beschrieben für die Vorwärmung der eingehenden Mischung eingesetzt wird, und zur Di-Nitrierung gefahren.

**Beispiel 2** soll ebenfalls zeigen, wie sich die einzelnen Massenströme zueinander verhalten können. Hier wird MNT an Stelle von Toluol als Aromat für die Umsetzung der Salpetersäure eingesetzt. Bei einem Nitrierprozess zur Herstellung von DNT fallen 24`951 kg/h Abfallsäure mit folgender Zusammensetzung an: 72 % H₂SO₄, 1 % HNO₃, 1,3 % HNO₂, 0,6 % DNT, 25,1 % H₂O. Weiterhin fallen 5`650 kg/h Waschwasser mit folgender Zusammensetzung an: 6 % H₂SO₄, 15 % HNO₃, 1 % HNO₂, 1 % DNT, 77 % H₂O. Aus der NOₓ Absorption werden 1'598 kg/h Salpetersäure mit 55 % HNO₃ und 45 % H₂O zurückgewonnen. Die 3 Ströme werden gemischt und auf 85 °C vorgewärmt. Anschließend werden zu der Mischung 50'033 kg/h ungekühlte rückkonzentrierte Schwefelsäure 1 mit 74 % H₂SO₄ und 26 % Wasser mit ca. 114 °C zugegeben, was der Siedetemperatur der Schwefelsäure in der zugehörigen Rückkonzentrierstufe bei 100 mbar entspricht. In die sich ergebende Mischung, die dann eine Temperatur von ca. 102,6 °C hat, werden 3'630 kg/h Toluol eingespritzt und die Mischung in den adiabatischen Rohrreaktor geleitet. Im adiabatischen Rohrreaktor wird die Mischung durch aufeinanderfolgende statische Mischer weiter durchmischt, um hier eine vollständige Reaktion zu erreichen. Bei dem Beispiel wurden 15 Mischelemente eingesetzt. Am Austritt des adiabatischen Reaktors erhält man eine Mischung mit 63,78 % H₂SO₄, 0,44 % HNO₂, 0,25 % MNT, 6,82 % DNT und 28,71 % H₂O. HNO₃ ist abreagiert und wenn überhaupt nur noch in Spuren vorhanden. Die Temperatur der Reaktionsmischung hat sich durch die frei werdende Reaktionsenergie und die adiabatische Reaktionsführung auf ca. 125 °C erhöht. Die Reaktionsmischung wird in einen Separator geleitet. An der Oberfläche scheidet sich die organische Phase ab und wird abgezogen. Die Schwefelsäurephase wird dann in einem Verdampfer bei 100 mbar gefälscht und durch die resultierende Wasserverdampfung entsprechend vorkonzentriert. Anschließend wird die Säure ebenfalls bei 100 mbar auf 74 % H₂SO₄ durch indirekte Beheizung, beispielsweise mit Dampf, nach den gängigen Methoden zur Schwefelsäurekonzentrierung entsprechend dem Stand der Technik weiter konzentriert. Die nicht zur adiabatischen Nitrierung zurückgefahrene Schwefelsäure wird im Vakuum von 50 mbar durch indirekte Beheizung mit Dampf nach den gängigen Methoden zur Schwefelsäurekonzentrierung entsprechend dem Stand der Technik weiter bis auf 94 % H₂SO₄ konzentriert.

## Patentansprüche

1. Verfahren zur Aufarbeitung von Mischsäure und Abwasser aus der Nitrierung von Aromaten, bei dem die enthaltene Salpetersäure durch Reaktion mit einem Aromaten unter adiabatischen Bedingungen umgesetzt wird, **dadurch gekennzeichnet, dass**
a. mindestens eine Abfallstromkomponente ausgewählt aus bei der Nitrierung anfallender Abfallsäure (Mischsäure), saurem Waschwasser aus der Aufarbeitung von Rohnitroaromaten und dünner Salpetersäure, die bei einer Abgasbehandlung im Verlauf der Nitrierung anfällt, bereitgestellt wird,
b. die mindestens eine Abfallstromkomponente mit rückkonzentrierter Schwefelsäure gemischt wird,
c. ein Aromat in stöchiometrischem Überschuss bezogen auf die Salpetersäure in die Mischung eindosiert wird,
d. die erhaltene Reaktionsmischung in einem adiabatisch betriebenen Reaktor umgesetzt wird,
e. in einem Separator die erhaltene organische Phase von der schwefelsauren Phase getrennt wird,
f. die schwefelsaure Phase im Vakuum aufkonzentriert wird, und
g. zumindest ein Teilstrom der rückkonzentrierten Schwefelsäure aus Schritt g) in Schritt b) eingesetzt wird.

2. Verfahren nach Anspruch 1, **dadurch gekennzeichnet, dass** in Schritt a) mindestens zwei, bevorzugt alle genannten Abfallstromkomponenten vermischt bereitgestellt werden..

3. Verfahren nach Anspruch 1 oder 2, **dadurch gekennzeichnet, dass** die mindestens eine Abfallstromkomponente in Schritt b) vor Zugabe der rückkonzentrierten Schwefelsäure vorgewärmt wird.

4. Verfahren nach Anspruch 3, **dadurch gekennzeichnet, dass** die Vorwärmung der Mischung so eingestellt wird, dass die Temperatur nach Zugabe der rückkonzentrierten Schwefelsäure im Bereich 70°C bis 130°C, bevorzugt im Bereich 90°C bis 110°C liegt.

5. Verfahren nach einem der Ansprüche 1 bis 4, **dadurch gekennzeichnet, dass** die Menge und Konzentration der rückkonzentrierten Schwefelsäure so gewählt wird, dass sich in Schritt b) eine Mischung mit einem Schwefelsäuregehalt zwischen 60 % und 70 % H₂SO₄ und einem Salpetersäuregehalt zwischen 1 % und 5 % HNO₃ ergibt.

6. Verfahren nach einem der Ansprüche 1 bis 5, **dadurch gekennzeichnet, dass** der Aromat in Schritt c) im stöchiometrischen Überschuss im Bereich 1 % - 20 % bevorzugt 3 % - 10 % bezogen auf die Salpetersäuremenge zugegeben wird.

7. Verfahren nach einem der Ansprüche 1 bis 6, **dadurch gekennzeichnet, dass** der Betriebsdruck am Reaktoreintritt im Bereich 1·10⁵ Pa bis 10·10⁵ Pa, bevorzugt im Bereich 3·10⁵ Pa bis 6·10⁵ Pa liegt.

8. Verfahren nach einem der Ansprüche 1 bis 7, **dadurch gekennzeichnet, dass** der Betriebsdruck im Separator zwischen 1·10⁵ Pa und 2·10⁵ Pa beträgt.

9. Verfahren nach einem der Ansprüche 1 bis 8, **dadurch gekennzeichnet, dass** sich die Reaktionsmischung im Reaktor durch die adiabatische Fahrweise um 10 bis 40 °C erhitzt.

10. Verfahren nach einem der Ansprüche 1 bis 9, **dadurch gekennzeichnet, dass** die schwefelsaure Phase im Vakuum bei einem Druck zwischen 30∗10² Pa und 500∗10² Pa aufkonzentriert wird.

11. Verfahren nach einem der Ansprüche 1 bis 10, **dadurch gekennzeichnet, dass** die nicht im Verfahren eingesetzte rückkonzentrierte Schwefelsäure bei einem Vakuum zwischen 150_{*}10² Pa und 30_{*}10² Pa bevorzugt zwischen 100_{*}10² Pa und 50_{*}10² Pa auf eine Endkonzentration zwischen 85 und 98 % H₂SO₄ weiterkonzentriert wird.

## Claims

1. Process for the workup of mixed acid and wastewater from the nitration of aromatics, in which the nitric acid present is reacted under adiabatic conditions by reaction with an aromatic, **characterised in that**
a. at least one waste stream component is provided, selected from waste acid (mixed acid) generated during the nitration, acidic washing water from the workup of crude nitroaromatics, and dilute nitric acid generated in an off-gas treatment in the course of the nitration,
b. the at least one waste stream component is mixed with re-concentrated sulfuric acid,
c. an aromatic is metered in to the mixture in stoichiometric excess relative to the nitric acid,
d. the reaction mixture obtained is reacted in an adiabatically operated reactor,
e. the organic phase obtained is separated from the sulfuric acid phase in a separator,
f. the sulfuric acid phase is concentrated under vacuum, and
g. at least one partial stream of the re-concentrated sulfuric acid from step g) is employed in step b).

2. Process according to claim 1, **characterised in that** at least two, preferably all, of the aforementioned waste stream components are provided mixed in step a).

3. Process according to claim 1 or claim 2, **characterised in that** the at least one waste stream component is preheated in step b) before the re-concentrated sulfuric acid is added.

4. Process according to claim 3, **characterised in that** the preheating of the mixture is adjusted in such a way that the temperature after the addition of the re-concentrated sulfuric acid is in the range from 70°C to 130°C, preferably in the range from 90°C to 110°C.

5. Process according to any of claims 1 to 4, **characterised in that** the amount and concentration of the re-concentrated sulfuric acid are selected in a manner that results, in step b), in a mixture having a sulfuric acid content of between 60% and 70% H₂SO₄ and a nitric acid content of between 1% and 5% HNO₃.

6. Process according to any of claims 1 to 5, **characterised in that**, in step c), the aromatic is added in stoichiometric excess in the range from 1%-20%, preferably 3%-10%, relative to the amount of nitric acid.

7. Process according to any of claims 1 to 6, **characterised in that** the operating pressure at the reactor inlet is in the range from 1×10⁵ Pa to 10×10⁵ Pa, preferably in the range from 3×10⁵ Pa to 6×10⁵ Pa.

8. Process according to any of claims 1 to 7, **characterised in that** the operating pressure in the separator is between 1×10⁵ Pa and 2×10⁵ Pa.

9. Process according to any of claims 1 to 8, **characterised in that** the reaction mixture in the reactor is heated by 10 to 40°C by the adiabatic procedure.

10. Process according to any of claims 1 to 9, **characterised in that** the sulfuric acid phase is concentrated under a vacuum at a pressure of between 30×10² Pa and 500×10² Pa.

11. Process according to any of claims 1 to 10, **characterised in that** the re-concentrated sulfuric acid which is not used in the process is further concentrated to a final concentration of between 85 and 98% H₂SO₄ under a vacuum of between 150×10² Pa and 30×10² Pa, preferably of between 100×10² Pa and 50×10² Pa.

## Revendications

1. Procédé de préparation d'acide mixte et d'eaux usées à partir de la nitration d'aromates, dans lequel l'acide nitrique obtenu est transformé par réaction avec un aromate dans des conditions adiabatiques, **caractérisé en ce que**
a. est fourni au moins un composant de flux résiduaire choisi parmi un acide résiduaire (acide mixte) résultant de la nitration, de l'eau de lavage acide issue de la préparation de nitro-aromates bruts et de l'acide nitrique dilué, qui résulte du traitement des effluents gazeux au cours de la nitration,
b. l'au moins un composant de flux résiduaire est mélangé à de l'acide sulfurique concentré,
c. un aromate en excédent stoechiométrique par rapport à l'acide nitrique est dosé dans le mélange,
d. le mélange réactionnel obtenu est transformé dans un réacteur à fonctionnement adiabatique,
e. la phase organique obtenue est séparée de la phase acide sulfurique dans un séparateur,
f. la phase acide sulfurique est concentrée sous vide, et
g. au moins un flux partiel de l'acide sulfurique concentré issu de l'étape g) est utilisé à l'étape b).

2. Procédé selon la revendication 1, **caractérisé en ce qu'**à l'étape a), au moins deux, de préférence tous les composants de flux résiduaire évoqués, sont fournis de manière mélangée.

3. Procédé selon la revendication 1 ou 2, **caractérisé en ce que** l'au moins un composant de flux résiduaire est préchauffé à l'étape b) avant l'ajout de l'acide sulfurique concentré.

4. Procédé selon la revendication 3, **caractérisé en ce que** le préchauffage du mélange est réglé de telle sorte que la température se situe, après l'ajout de l'acide sulfurique concentré, dans la plage de 70 °C à 130 °C, de manière préférée dans la plage de 90 °C à 110 °C.

5. Procédé selon l'une quelconque des revendications 1 à 4, **caractérisé en ce que** la quantité et la concentration de l'acide sulfurique concentré sont choisies de telle sorte qu'il découle, à l'étape b), un mélange avec une teneur en acide sulfurique entre 60 % et 70 % de H₂SO₄ et une teneur en acide nitrique entre 1 % et 5 % de HNO₃.

6. Procédé selon l'une quelconque des revendications 1 à 5, **caractérisé en ce que** l'aromate est ajouté à l'étape c) en excédent stoechiométrique dans la plage de 1 % - 20 %, de manière préférée de 3 % - 10 % par rapport à la quantité d'acide nitrique.

7. Procédé selon l'une quelconque des revendications 1 à 6, **caractérisé en ce que** la pression de fonctionnement à l'entrée de réacteur se situe dans la plage de 1·10⁵ Pa à 10·10⁵ Pa, de manière préférée dans la plage de 3·10⁵ Pa à 6·10⁵ Pa.

8. Procédé selon l'une quelconque des revendications 1 à 7, **caractérisé en ce que** la pression de fonctionnement dans le séparateur est comprise entre 1·10⁵ Pa et 2·10⁵ Pa.

9. Procédé selon l'une quelconque des revendications 1 à 8, **caractérisé en ce que** le mélange réactionnel dans le réacteur se réchauffe de 10 à 40 °C par le mode opératoire adiabatique.

10. Procédé selon l'une quelconque des revendications 1 à 9, **caractérisé en ce que** la phase acide sulfurique est concentrée sous vide à une pression entre 30^{∗}10² Pa et 500^{∗}10² Pa.

11. Procédé selon l'une quelconque des revendications 1 à 10, **caractérisé en ce que** l'acide sulfurique concentré non utilisé dans le procédé est concentré ultérieurement sous vide entre 150^{∗}10² Pa et 30^{∗}10² Pa, de manière préférée entre 100^{∗}10² Pa et 50^{∗}10² Pa sur une concentration finale entre 85 et 98 % de H₂SO₄.
